# EUROPÄISCHE PATENTSCHRIFT

(11) **EP 3 563 899 B1**
(45) Veröffentlichungstag und Bekanntmachung des Hinweises auf die Patenterteilung: **27.08.2025**
(21) Anmeldenummer: 19168918.1
(22) Anmeldetag: 12.04.2019
(51) Int. Cl.: A61M 25/10, A61B 17/22

(54) **KATHETEREINRICHTUNG FÜR EINE CTO-REKANALISIERUNG**
CATHETER DEVICE FOR CTO RECANALISATION
DISPOSITIF CATHÉTER POUR UNE RECANALISATION D'UNE OCCLUSION CORONAIRE TOTALE CHRONIQUE OCT

(30) Priorität: 03.05.2018 EP 18170549
(43) Veröffentlichungstag der Anmeldung: 06.11.2019
(73) Patentinhaber: BIOTRONIK AG, 8180 Bülach (CH)
(72) Erfinder: Fargahi, Amir, 8180 Bülach (CH)
(74) Vertreter: WSL Patentanwälte Partnerschaft mbB

(56) Entgegenhaltungen:
- WO-A1-2007/100365
- WO-A1-95/16487
- WO-A2-99/07354
- US-A1- 2006 271 090

## Beschreibung

Die Erfindung betrifft eine Kathetereinrichtung (1) zum Rekanalisieren einer Okklusion (O) in einem Gefäß (G), mit einem am distalen Endabschnitt (2a) angeordneten proximalen Ballon (3) zum Verankern des Katheterschafts (2) in dem Gefäß (G) sowie zum Aufweiten der Okklusion (O) und einem am distalen Endabschnitt (2a) angeordneten distalen Ballon (4) zum Aufweiten der Okklusion (O).

Derartige Kathetereinrichtungen werden insbesondere zum Rekanalisieren bzw. Wiedereröffnen eines chronischen, im Wesentlichen vollständigen Verschlusses (Chronic Total Occlusion oder kurz CTO) eines Gefäßes eines Patienten verwendet.

Derartige CTOs kommen bei ca. 20% aller Patienten im Herzkatheter-Labor vor. Die durchschnittliche Interventionsdauer liegt in der Regel bei etwa zwei bis 2,5 Stunden. Die Anzahl der verwendeten Führungsdrähte, Ballon-Katheter und Stents ist dabei regelmäßig größer als bei typischen perkutanen Koronarinterventionen (PCI), die keinen CTO betreffen.

Zur Behandlung bzw. Rekanalisierung von chronischen Totalverschlüssen (CTOs) ist aus dem Stand der Technik gemäß WO2007/100365A1 z.B. eine Kathetereinrichtung bekannt, die zwei hintereinander angeordnete Ballons aufweist, durch die sich ein Führungsdraht hindurch erstreckt, der zum Positionieren der beiden Ballons vor der Okklusion verwendet wird, wobei der Führungsdraht zunächst durch den Verschluss geführt wird. In US20060271090A1 wird ebenfalls ein Doppelballonsystem offenbart. In WO1995016487A1 wird ein diagnostisches Kathetersystem und in WO9907354A2 werden Therapieformen zur Auslösung einer ventrikulären Asystole offenbart.

Bei derartigen Systemen kann sich das zum Aufnehmen des Führungsdrahts in den beiden Ballons vorgesehene Führungsdrahtlumen als nachteilig erweisen, da hierfür ein entsprechender Bauraum vorgesehen werden muss, so dass die Einrichtung einen vergleichsweise großen Durchmesser im Bereich der Ballone aufweist.

Ferner kommt es bei derartigen Ballonsystemen regelmäßig zu Rückstoßeffekten während der Balloninflation, da der führende (distale Ballon) beim Entfalten/Dilatieren im Durchmesser zunimmt und daher tendenziell aus der verengten Stelle herausgedrückt wird.

Hiervon ausgehend liegt der vorliegenden Erfindung daher die Aufgabe zugrunde, eine Kathetereinrichtung bereitzustellen, die hinsichtlich der oben geschilderten Problematik verbessert ist.

Diese Aufgabe wird durch eine Kathetereinrichtung mit den Merkmalen des Anspruchs 1 gelöst.

Vorteilhafte Ausgestaltungen sind in den Unteransprüchen angegeben und werden nachfolgend beschrieben.

Demgemäß wird eine Kathetereinrichtung zum Rekanalisieren einer Okklusion (insbesondere CTO) in einem Gefäß vorgeschlagen, mit:
- einem entlang einer Längsachse erstreckten Katheterschaft, der einen distalen Endabschnitt aufweist,
- einem am distalen Endabschnitt angeordneten proximalen Ballon zum Verankern des Katheterschafts in dem Gefäß sowie zum Aufweiten der Okklusion, der am distalen Endabschnitt des Katheterschafts angeordnet ist, wobei der proximale Ballon einen Balloninnenraum aufweist, in den zum Entfalten des proximalen Ballons ein fluides Medium einleitbar ist,
- einem am distalen Endabschnitt angeordneten distalen Ballon zum initialen Aufweiten der Okklusion, wobei der distale Ballon einen Balloninnenraum aufweist, in den zum Entfalten des proximalen Ballons ein fluides Medium einleitbar ist, und
- einem vom Katheterschaft umgebenen Führungsdrahtlumen sowie einem im Führungsdrahtlumen führbaren Führungsdraht, wobei der Katheterschaft am distalen Endabschnitt eine mit dem Führungsdrahtlumen kommunizierende Öffnung aufweist, über die der Führungsdraht aus dem Führungsdrahtlumen sowie aus dem Katheterschaft herausführbar ist.

Erfindungsgemäß ist nun vorgesehen, dass die Öffnung so angeordnet ist, dass der Führungsdraht mit einem über die Öffnung aus dem Führungsdrahtlumen herausgeführten Abschnitt des Führungsdrahtes außerhalb des distalen Ballons verläuft sowie insbesondere außen am distalen Ballon vorbeiführbar ist, so dass der Führungsdraht insbesondere am distalen Ballon vorbei in die Okklusion einführbar ist, um den Katheterschaft insbesondere bezüglich der Okklusion zu positionieren. Das Führungsdrahtlumen durchläuft dabei den distalen Endabschnitt, wodurch das Führungsdrahtlumen innerhalb des proximalen Ballons bzw durch den proximalen Ballon verläuft, aber nicht den distalen Ballon durchläuft.

Gemäß einer Ausführungsform ist diesbezüglich vorgesehen, dass die Öffnung entlang Längsachse des Katheterschafts zwischen dem Balloninnenraum des proximalen Ballons und dem Balloninnenraum des distalen Ballons angeordnet ist. Insbesondere kann die Öffnung entlang der besagten Längsachse am distalen Ende des proximalen Ballons vorgesehen sein oder zwischen dem distalen Ende des proximalen Ballons und einem proximalen Ende des distalen Ballons. Die Öffnung ist dabei bevorzugt im oder nah am axialen Zentrum des proximalen Ballon angeordnet. Die Öffnung ist daher nicht in der Peripherie oder an der Außenseite des proximalen Ballons angeordnet. Eine solche Anordnung ermöglicht es sowohl über die Verlängerung des Führungsdrahtes über die Öffnung hinaus mit dem Führungsdraht als auch durch das Führungselement die Okklusion zu öffnen.

Wenn im Rahmen der vorliegenden Erfindung von einer proximalen Komponente oder einem proximalen Bereich und einer entsprechenden distalen Komponente bzw. einem distalen Bereich die Rede ist, so bedeutet das, dass die distale Komponente bzw. der distale Bereich entlang des Verlaufs des Katheterschafts weiter von einem Bediener der Kathetereinrichtung entfernt ist, als die proximale Komponente bzw. der proximale Bereich. Bei dem Bereich kann es sich z.B. um einen Endabschnitt oder ein Ende einer Komponente der Kathetereinrichtung handeln.

Aufgrund der erfindungsgemäßen Gestaltung der Kathetereinrichtung besteht die Möglichkeit, den distalen Ballon verhältnismäßig klein auszugestalten, da dieser das Führungsdrahtlumen nicht mehr beherbergen muss.

Der distale Ballon weist gemäß einer Ausführungsform der Erfindung vorzugsweise einen Durchmesser auf, der im entfalteten Zustand des distalen Ballons wesentlich kleiner ist als der Durchmesser des entfalteten proximalen Ballons. Der distale Ballon wird daher auch als Mikroballon bezeichnet. Der Durchmesser des proximalen Ballons ist vorzugsweise insbesondere so ausgelegt, dass sich der proximale Ballon in einem entfalteten Zustand an die Gefäßwand des Patienten zum Verankern des Katheterschafts anlegen kann. In besonderen Ausführungsformen ist der Durchmesser des proximalen Ballon im entfalteten Zustand vorzugsweise mehr als doppelt so groß, weiter bevorzugt mehr als 4-fach so groß und am meisten bevorzugt mehr als 10-fach so groß wie der Durchmesser der distalen Ballons im entfalteten Zustand. Ferner ist es bevorzugt, wenn der distale Ballon rigider ausgelegt ist als der proximale Ballon.

Zum Rekanalisieren der CTO kann die Kathetereinrichtung bzw. der Katheterschaft über den Führungsdraht in den Körper bzw. das betroffene Gefäß des Patienten eingeführt und positioniert werden. Danach wird der Führungsdraht zurückgezogen und der distale Ballon wird in die Okklusion geführt. Die gleichzeitige Dilatation des distalen bzw. Mikroballons sowie des proximalen Ballons sprengt die Okklusion am Eingang. In weiteren Schritten wird der proximale Ballon deflatiert, vorwärts in die Okklusion eingeführt und wieder dilatiert, bis eine vollständige Öffnung des Gefäßes bzw. eine Rekanalisierung der Okklusion erreicht worden ist.

Mittels der erfindungsgemäßen Einrichtung wird eine CTO-Rekanalisierung vereinfacht, da der distale Ballon verhältnismäßig dünn ist und entsprechend leichter in die Okklusion bewegbar ist. Weiterhin kann ein beim Dilatieren des distalen Ballons erzeugter Rückstoß gut mit dem proximalen Ballon aufgefangen werden, da dieser auf der Innenwand des Gefäßes verankerbar ist (siehe oben).

Weiterhin ist gemäß einer Ausführungsform der Erfindung vorgesehen, dass die Kathetereinrichtung ein zu dem Führungsdraht separat ausgebildetes längserstrecktes Führungselement aufweist, das mit einem distalen Endabschnitt von dem zweiten Ballon in distaler Richtung absteht.

Weiterhin ist gemäß einer Ausführungsform der Erfindung vorgesehen, dass sich das Führungselement durch den Balloninnenraum des distalen Ballons hindurch erstreckt, wobei jener distale Endabschnitt des Führungselements an einem distalen Ende des distalen Ballons aus dem distalen Ballon herausgeführt ist.

Gemäß einer Ausführungsform der Erfindung kann das Führungselement z.B. durch einen Draht gebildet ist sein. Der Draht kann einen röntgensichtbaren Kern aufweisen, der aus Platin oder aus einer Platinlegierung geformt ist, wobei der Kern mit einer Cobalt-Chrom-Legierung beschichtet sein kann. Weiterhin kann das Führungselement bzw. der Draht einen Röntgenmarker aufweisen. Weiterhin ist in einer Ausführungsform das Führungselement von der Spitze des distalen Ballons zum aus dem Führungsdrahtlumen herausgeführten Abschnitt, in dem der Führungsdraht außerhalb des distalen Ballons distal zur Öffnung des distalen Endabschnittes verlaufen kann, hingeneigt.

Weiterhin ist gemäß einer Ausführungsform der Erfindung vorgesehen, dass das Führungsdrahtlumen des Katheterschafts von einer Wandung umgeben ist. Hierbei ist gemäß einer Ausführungsform vorgesehen, dass sich die Wandung des Führungsdrahtlumens durch den Balloninnenraum des proximalen Ballons hindurch erstreckt.

Weiterhin ist gemäß einer Ausführungsform der Erfindung vorgesehen, dass der Katheterschaft ein von einer Wandung umgebenes Lumen (Inflationslumen) aufweist, über das zum, insbesondere gleichzeitigen, Entfalten des proximalen und des distalen Ballons ein fluides Medium in den Balloninnenraum des proximalen Ballons und in den Balloninnenraum des distalen Ballons einleitbar ist. Es ist also vorgesehen, dass der proximale Ballon und der distale Ballon über ein einzelnes (Inflations)lumen mit einem fluiden Medium inflatierbar sind.

Gemäß einer Ausführungsform der Erfindung ist vorgesehen, dass das Lumen in den Balloninnenraum des proximalen Ballons mündet, wobei der Balloninnenraum des distalen Ballons über den Balloninnenraum des proximalen Ballons mit dem Lumen in Strömungsverbindung steht.

Ein solcher Aufbau hat den Vorteil, dass im dem Moment, in dem man das Führungselement und den distalen Ballon weiter in die Okklusion einführen möchte, das Verankern im Gefäß durch den proximalen Ballon gelöst werden muss, wodurch auch der distale Ballon, der in die Okklusion eingeführt werden soll, an Volumen verliert und leichter vorgeschoben werden kann. Es ist daher nicht nötig, erst die Verankerung durch den proximalen Ballon zu lösen und danach das Volumen des distalen Ballon einzustellen wie dies bei einer Ausgestaltung notwendig wäre, bei der separate (Inflations)lumen für den distalen und den proximalen Ballon vorliegen.

Weiterhin ist gemäß einer Ausführungsform der Erfindung vorgesehen, dass sich die Wandung des Lumens durch den Balloninnenraum des proximalen Ballons hindurch erstreckt, wobei das Lumen mit dem Balloninnenraum des distalen Ballons in Strömungsverbindung steht, und wobei die Wandung des Lumens zumindest eine im Balloninnerraum des proximalen Ballons angeordnete laterale Auslassöffnung aufweist, über die ein fluides Medium zum Entfalten des proximalen Ballons in den Balloninnenraum des proximalen Ballons einleitbar ist.

Weiterhin ist gemäß einer Ausführungsform vorgesehen, dass ein proximaler Endabschnitt des Führungselements mit der Wandung des Führungsdrahtlumens verbunden ist. Dies gilt insbesondere für den Fall, dass sich das Lumen nicht durch den Balloninnenraum des proximalen Ballons hindurch erstreckt bzw. nicht vollständig bis zum distalen Ballon erstreckt.

Gemäß einer alternativen Ausführungsform der Erfindung ist vorgesehen, dass der proximale Endabschnitt des Führungselements mit der Wandung des Lumens verbunden ist. Dies kann insbesondere der Fall ein, wenn sich das besagte Lumen bzw. die Wandung des Lumens durch den Balloninnenraum des proximalen Ballons hindurch erstreckt.

Weiterhin ist gemäß einer Ausführungsform der Erfindung vorgesehen, dass ein proximales Ende des distalen Ballons mit der Wandung des Führungsdrahtlumens und/oder mit der Wandung des Lumens verbunden ist.

Weiterhin ist gemäß einer Ausführungsform der Erfindung vorgesehen, dass sich das Lumen und das Führungsdrahtlumen nebeneinander im Katheterschaft entlang der Längsachse erstrecken.

Gemäß einer alternativen Ausführungsform kann ebenfalls vorgesehen sein, dass das Lumen und das Führungsdrahtlumen koaxial zueinander angeordnet sind, wobei das Führungsdrahtlumen von dem besagten Lumen umgeben ist. In diesem Fall umschließt die Wandung des Lumens die Wandung des Führungsdrahtlumens.

Gemäß einer weiteren Ausführungsform der Erfindung ist vorgesehen, dass die Kathetereinrichtung mindestens einen Röntgenmarker für den proximalen Ballon aufweist, der im Balloninnenraum des proximalen Ballons an der Wandung des Führungsdrahtlumens angeordnet ist.

Weitere Merkmale und Vorteile der Erfindung sollen bei der Figurenbeschreibung von Ausführungsbeispielen der Erfindung anhand der Figuren erläutert werden. Es zeigen:
- Fig. 1: eine schematische Schnittansicht einer Ausführungsform einer erfindungsgemäßen Kathetereinrichtung;
- Fig. 2: eine schematische Schnittansicht einer weiteren Ausführungsform einer erfindungsgemäßen Kathetereinrichtung;
- Fig. 3: eine schematische Schnittansicht einer weiteren Ausführungsform einer erfindungsgemäßen Kathetereinrichtung;
- Fig. 4: eine Schnittdarstellung einer Ausführungsform des Katheterschafts der Kathetereinrichtung;
- Fig. 5: eine Schnittdarstellung einer alternativen Ausführungsform des Katheterschafts der Kathetereinrichtung; und
- Fig. 6: zeigt eine mögliche Rekanalisierung einer totalen Okklusion (CTO) unter Verwendung einer erfindungsgemäßen Kathetereinrichtung.

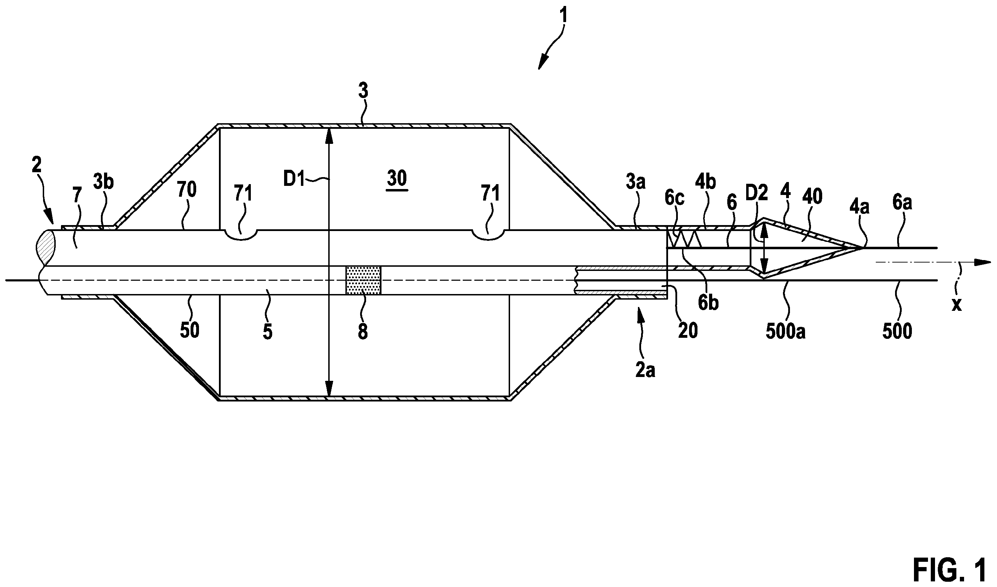

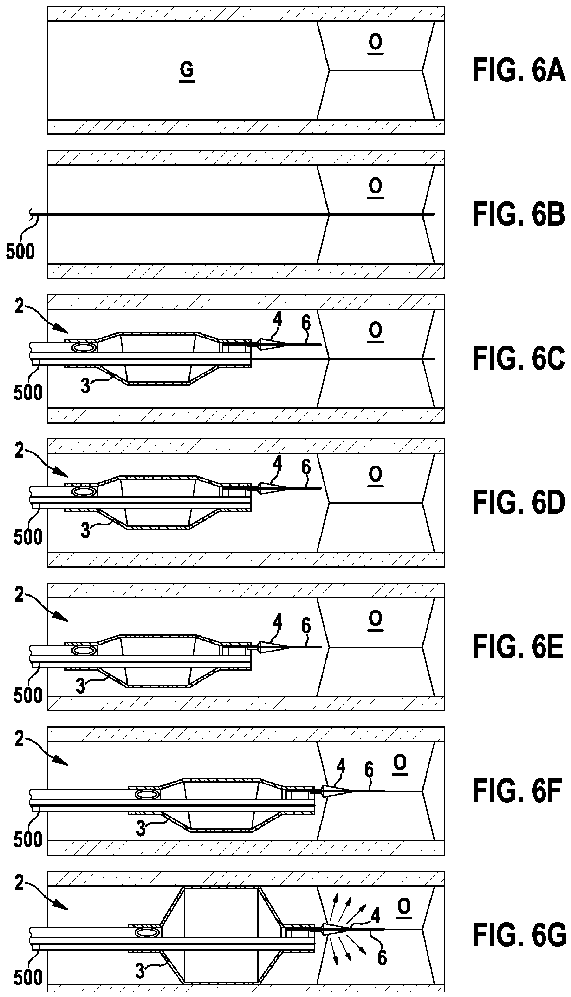

Die Figuren 1 bis 3 zeigen Ausführungsformen einer erfindungsgemäßen Kathetereinrichtung 1 zum Rekanalisieren einer Okklusion O in einem Gefäß G (vgl. Figuren 6(A) bis 6(M)). Die Kathetereinrichtung weist danach einen entlang einer Längsachse x erstreckten Katheterschaft 2 auf, der einen distalen Endabschnitt 2a aufweist, an dem ein proximaler Ballon 3 zum Verankern des Katheterschafts 2 in dem Gefäß G angeordnet ist, der ferner zum Aufweiten der Okklusion O konfiguriert ist, wobei der proximale Ballon 3 einen Balloninnenraum 30 aufweist, in den zum Entfalten des proximalen Ballons 3 ein fluides Medium (z.B. physiologische Kochsalzlösung, Kontrastmittel) einleitbar ist. Die Kathetereinrichtung 1 weist weiterhin einen am distalen Endabschnitt 2a angeordneten distalen Ballon 4 zum initialen Aufweiten der Okklusion O auf, wobei der distale Ballon 4 ebenfalls einen Balloninnenraum 40 aufweist, in den zum Entfalten des proximalen Ballons 3 ebenfalls ein fluides Medium (z.B. physiologische Kochsalzlösung, Kontrastmittel) einleitbar ist. Die Einrichtung 1 weist weiterhin ein vom Katheterschaft 2 gebildetes Führungsdrahtlumen 5 auf sowie einen im Führungsdrahtlumen 5 führbaren Führungsdraht 500, wobei der Katheterschaft 2 am distalen Endabschnitt 2a eine Öffnung 20 aufweist, über die der Führungsdraht 500 aus dem Führungsdrahtlumen 5 sowie aus dem Katheterschaft 2 herausführbar ist.

Erfindungsgemäß ist vorgesehen, dass die Öffnung 20 so angeordnet ist, dass der Führungsdraht 500 mit einem über die Öffnung 20 aus dem Führungsdrahtlumen 5 herausgeführten Abschnitt 500a des Führungsdrahtes 500 außerhalb des distalen Ballons 4 verläuft und dabei insbesondere an dem distalen Ballon vorbei in die Okklusion einführbar ist, um den Katheterschaft 2 zum Zielort zu führen.

Insbesondere ist die Öffnung 20 entlang der Längsachse x des Katheterschafts 2 zwischen einem distalen Ende 3a des proximalen Ballons und einem proximalen Ende 4b des distalen Ballons 4 angeordnet.

Der Führungsdraht 500 kann beispielsweise einen Durchmesser von 0,36mm aufweisen, wobei der Innendurchmesser des Führungsdrahtlumens 5 größer ausgestaltet ist, so dass der Führungsdraht 500 gut im Führungsdrahtlumen 5 gleiten kann.

Zum Einführen bzw. Positionieren des distalen Ballons 4 in die Okklusion O weist die Kathetereinrichtung 1 vorzugsweise ein zu dem Führungsdraht 500 separat ausgebildetes längserstrecktes Führungselement 6 aufweist, das insbesondere röntgensichtbar ausgebildet und das mit einem distalen Endabschnitt 6a von dem distalen Ballon 4 absteht. Gemäß einer Ausführungsform kann der distale Endabschnitt 6a eine Länge (entlang der Längsachse x) im Bereich von 2 mm bis 10 mm aufweisen. Das Führungselement 6 erstreckt sich insbesondere entlang der Längsachs x durch den Balloninnenraum 40 des distalen Ballons 4 hindurch, wobei jener distale Endabschnitt 6a des Führungselements 6 aus dem distalen Ballon 4 an einem distalen Ende 4a des distalen Ballons 4 aus dem distalen Ballon 4 herausgeführt ist.

Weiterhin kann der Draht bzw. das Führungselement 6 gemäß einer Ausführungsform einen Durchmesser von 0,1mm aufweisen.

Weiterhin kann der distale Ballon 4 gemäß einer Ausführungsform bei 6 atm einen Durchmesser D2 im Bereich von 1,0 mm bis 2,0 mm aufweisen. Diesbezüglich ist weiterhin vorgesehen, dass der Durchmesser D2 kleiner ist als der Durchmesser D1 des vollständig dilatierten bzw. entfalteten proximalen Ballons 3.

Weiterhin kann der distale Ballon 4 gemäß einer Ausführungsform entlang der Längsachse x eine Länge im Bereich von 2 mm bis 10 mm aufweisen.

Weiterhin kann eine doppelte Wandstärke des distalen Ballons 4 gemäß einer Ausführungsform im Bereich von 0,02 mm bis 0,04 mm liegen. Der Profildurchmesser des distalen Ballons 4 kann des Weiteren gemäß einer Ausführungsform (gefaltet auf das Führungselement 6 das einen Durchmesser von 0,1mm aufweist) kleiner oder gleich 0,2 mm sein.

Weiterhin kann der distale Ballon 4 gemäß einer Ausführungsform aus einem der folgenden Materialien gefertigt sein: Polyamid, insbesondere PA12, Polyethylenterephthalat, Polyurethan oder Polyether Blockamide wie Pebax (Markenname). Weiterhin kann der distale Ballon eine Beschichtung auf seiner Außenseite aufweisen, die hydrophil oder hydrophob ist.

Weiterhin ist vorgesehen, dass das Führungsdrahtlumen 5 des Katheterschafts 2 von einer Wandung 50 umgeben ist. Zum Verfolgen des proximalen Ballons 3 kann des Weiteren vorgesehen sein, dass die Kathetereinrichtung 1 mindestens einen Röntgenmarker 8 aufweist, der im Balloninnenraum 30 des proximalen Ballons 3 an einer Wandung 50 angeordnet ist, die das Führungsdrahtlumen 5 umgibt. Weiterhin ist vorgesehen, dass sich die Wandung 50 des Führungsdrahtlumens 5 durch den Balloninnenraum 30 des proximalen Ballons 3 entlang der Längsachse x hindurch erstreckt und dabei stirnseitig die besagte Öffnung 20 des distalen Endabschnitts 2a des Katheterschafts 2 berandet.

Weiterhin weist der Katheterschaft gemäß den Figuren 1 bis 3 ein von einer Wandung 70 umgebenes Lumen 7 auf, über das zum Entfalten des proximalen und des distalen Ballons 3, 4 ein fluides Medium, z.B. physiologische Kochsalzlösung oder Kontrastmittel, in den Balloninnenraum 30 des proximalen Ballons 3 und in den Balloninnenraum 40 des distalen Ballons 4 einleitbar ist.

Gemäß der in der Figur 1 gezeigten Ausführungsform ist vorgesehen, dass sich die Wandung 70 des Lumens 7 entlang der Längsachse x durch den Balloninnenraum 30 des proximalen Ballons 3 hindurch erstreckt, wobei das Lumen 7 mit dem Balloninnenraum 40 des distalen Ballons 4 in Strömungsverbindung steht bzw. in den Balloninnenraum 40 mündet, so dass der distale Ballon 4 über das Lumen 7 dilatierbar ist. Zum Dilatieren des proximalen Ballons 3 weist die Wandung 70 des Lumens 7 ferner im Balloninnerraum 30 des proximalen Ballons 3 angeordnete laterale Auslassöffnungen 71 auf, so dass das fluide Medium über die Auslassöffnung 71 zum Entfalten des proximalen Ballons 3 auch in den Balloninnenraum 30 des proximalen Ballons 3 einleitbar ist.

Weiterhin ist gemäß Figur 1 vorgesehen, dass ein proximaler Endabschnitt 6b des sich durch den Balloninnenraum 40 des distalen Ballons 4 erstreckende Führungselement 6 an der Wandung 70 des Lumens 7 über eine z.B. stoffschlüssige Verbindung 6c festgelegt ist.

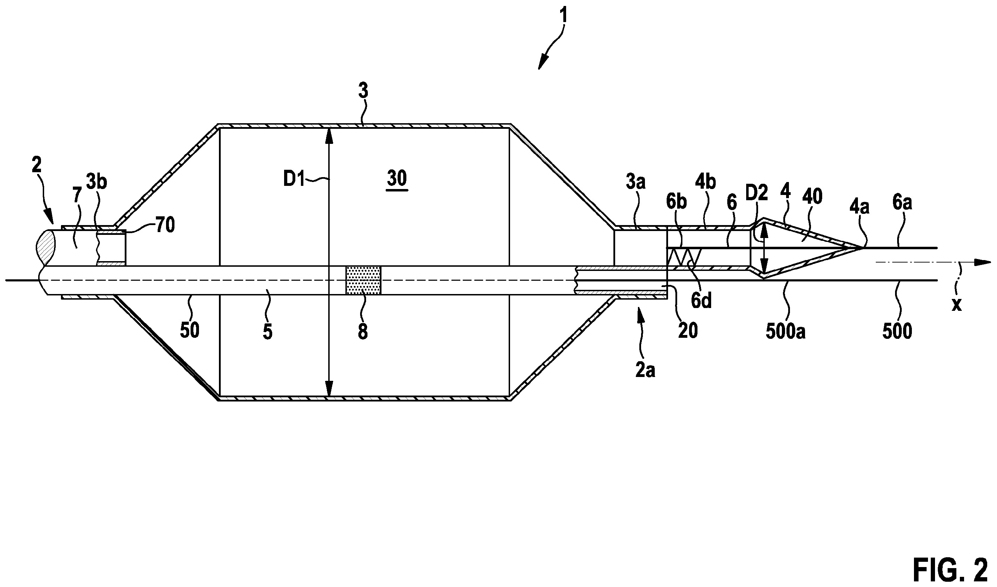

Figur 2 zeigt demgegenüber eine alternative Ausführungsform, wobei hier die Wandung 70 des Lumens 7 am proximalen Ende 3b des proximalen Ballons endet bzw. das Lumen 7 in den Balloninnenraum 30 des proximalen Ballons 3 mündet. Der Balloninnenraum 30 des proximalen Ballons 3 steht dabei mit dem Balloninnenraum 40 des distalen Ballons 4 in Strömungsverbindung, so dass der distale Ballon 4 über den proximalen Ballon 3 dilatierbar bzw. inflatierbar ist. Bei der Ausführungsform gemäß Figur 2 ist im Unterschied zur Figur 1 vorgesehen, dass der proximale Endabschnitt 6b des Führungselements 6 mit der Wandung 50 des Führungsdrahtlumens 5 über eine z.B. stoffschlüssige Verbindung 6d verbunden ist.

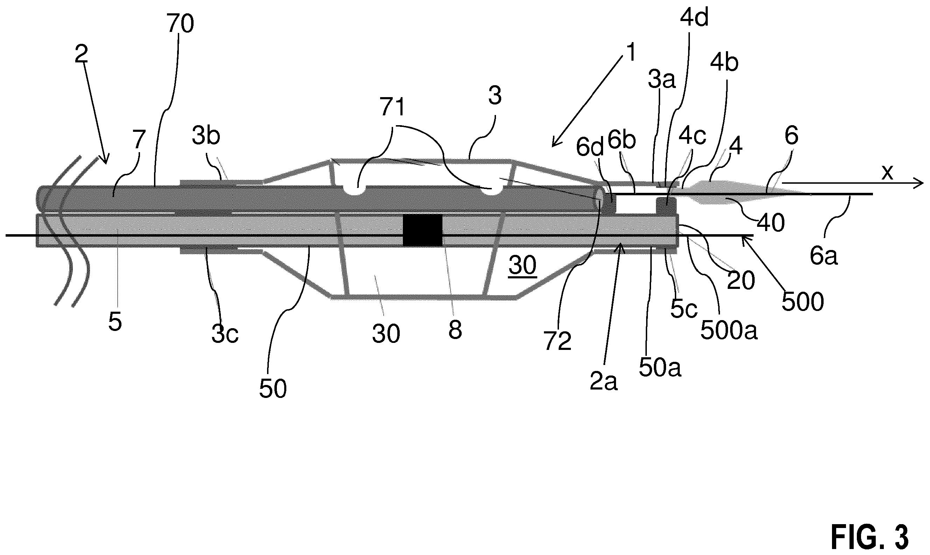

Figur 3 zeigt eine weitere Ausführungsform einer erfindungsgemäßen Kathetereinrichtung, wobei sich hier die Wandung 70 des Lumens 7 ebenfalls durch den Balloninnenraum 30 des proximalen Ballons 3 hindurch erstreckt, jedoch entlang der Längsachse x vor dem distalen Ende 3a des proximalen Ballons 3 endet, wobei eine stirnseitige Auslassöffnung 72 der Wandung 70 dem distalen Ballon 4 zugewandt ist und mit diesem über das umlaufende distale Ende 3a des proximalen Ballons 3 strömungstechnisch verbunden ist. Der proximale Endabschnitt 6b des Führungselements/Drahts 6 steht hierbei in das distale Ende 3a des proximalen Ballons 3 hinein und ist über eine z.B. stoffschlüssige Verbindung 6d mit der Wandung 50 des Führungsdrahtlumens 5 verbunden.

Weiterhin ist insbesondere vorgesehen, dass ein proximales Ende 4b des distalen Ballons 4 einerseits über eine z.B. stoffschlüssige Verbindung 4d mit dem distalen Ende 3a des proximalen Ballons 3 verbunden ist sowie andererseits über eine z.B. stoffschlüssige Verbindung 4c mit einem distalen Ende 50a der Wandung 50 des Führungsdrahtlumens 5. Das distale Ende 50a der Wandung 50 des Führungsdrahtlumens kann des Weiteren über eine z.B. stoffschlüssige Verbindung 5c mit dem distalen Ende 3a des proximalen Ballons 3 verbunden sein. Schließlich ist insbesondere das proximale Ende 3b des proximalen Ballons 3 über eine z.B. stoffschlüssige Verbindung 3c mit dem Schaft 2 bzw. der Wandung 50 des Führungsdrahtlumens und/oder der Wandung 70 des Lumens 7 verbunden.

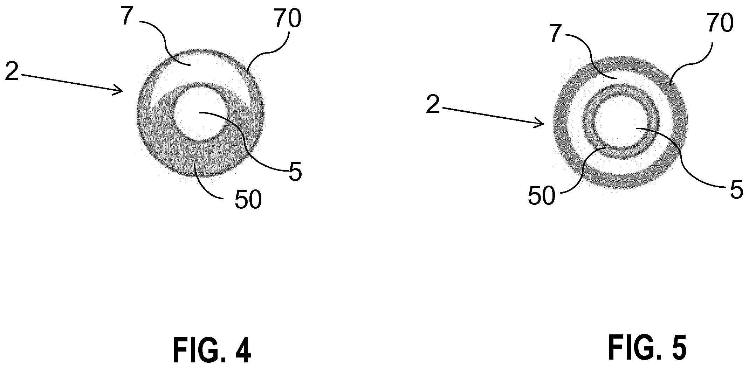

Der Katheterschaft 2 kann auf verschiedene Weisen das Lumen 7 bzw. das Führungsdrahtlumen 5 umschließen bzw. begrenzen. Die Figuren 4 und 5 zeigen zwei prinzipielle Möglichkeiten, die beide bei den in den Figuren 1 bis 3 gezeigten Ausführungsformen verwendet werden können. Gemäß Figur 4 können das Führungsdrahtlumen 5 und das Lumen 7 benachbart nebeneinander her im Katheterschaft 2 erstreckt sein. Die beiden Wandungen 50 bzw. 70 sind entsprechend entlang der Längsachse x miteinander verbunden oder integral miteinander ausgebildet.

Alternativ hierzu können das Lumen 7 und das Führungsdrahtlumen 5 gemäß Figur 5 koaxial zueinander angeordnet sein, wobei das Führungsdrahtlumen 5 vom Lumen 7 umgeben ist. Die Wandung 50 des Führungsdrahtlumens 5 erstreckt sich hierbei in dem von der äußeren Wandung 70 umgebenden Lumen 7.

Die Figuren 6(A) bis 6(M) zeigen exemplarisch die Verwendung einer erfindungsgemäßen Kathetereinrichtung 1 (z.B. nach Art der Figuren 1 bis 3) zum Rekanalisieren einer totalen Okklusion O eines Gefäßes G.

Nach initialer Beurteilung der Verengung (CTO) O (vgl. Fig. 6(A)) wird der Führungsdraht 500 der Kathetereinrichtung 1 durch die Okklusion O hindurchgeführt (vgl. Fig. 6(B)). Mittels des Führungsdrahts 500 wird der Katheterschaft 2 der Einrichtung 1 sodann in dem Gefäß bzw. der Arterie G positioniert (vgl. Fig. 6(C)).

Nach dem Positionieren des Katheterschafts 2 wird der Führungsdraht 500 zurückgezogen (vgl. Fig. 6(D)) und der distale Ballon 4 wird bezüglich der Okklusion O positioniert (vgl. Fig. 6(E)) und in diese eingeführt (vgl. Fig. 6(F)).

Sodann werden der proximale Ballon 3 und der distale Ballon 4 inflatiert, um die Okklusion O aufzuweiten (vgl. Fig. 6(G)). Hierbei dient der proximale Ballon 3 zum Verankern des Katheterschafts 2 im Gefäß G.

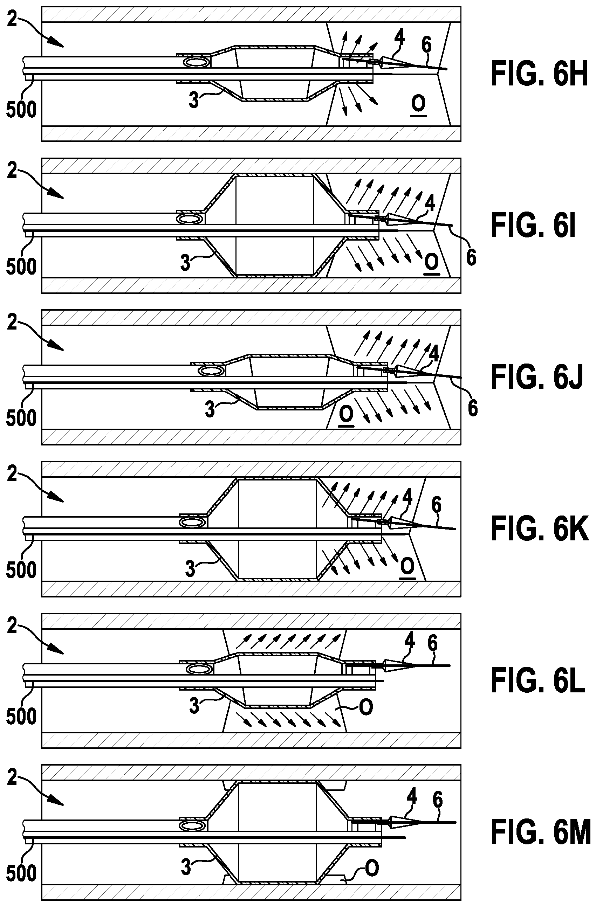

Hiernach werden die beiden Ballons 3, 4 deflatiert und teilweise über den Führungsdraht 500 und das längserstreckte Führungselement 6 in die Verengung O einführt (vgl. Fig. 6(H)). Anschließend werden die beiden Ballons 3, 4 dilatiert, wobei der distale Ballon 4 die Okklusion O stärker aufweitet und der proximale Ballon 3 wiederum für eine Verankerung des Schafts 2 im Gefäß G sorgt (vgl. Fig. 6(I)).

Anschließend werden gemäß Fig. 6(J) die beiden Ballons 3, 4 deflatiert und weiter in die Okklusion O eingeführt. Hiernach werden die beiden Ballons 3, 4 wieder dilatiert um die Läsion O noch stärker aufzuweiten (vgl. Fig. 6(K)).

Anschließend werden gemäß Fig. 6(L) beide Ballons 3, 4 deflatiert und er proximale Ballon 3 wird mittig in die Okklusion O eingeführt und sodann dilatiert (vgl. Fig. 6(M)) um das Gefäß G an der Stelle der Okklusion O auf den Enddurchmesser aufzuweiten.

Die erfindungsgemäße Einrichtung 1 ermöglicht mit Vorteil einer Rekanalisierung einer CTO mittels einer einzigen Einrichtung 1, wodurch beim Eingriff Zeit gespart wird und das Risiko für den Patienten entsprechend herabgesetzt wird.

## Patentansprüche

1. Kathetereinrichtung (1) zum Rekanalisieren einer Okklusion (O) in einem Gefäß (G), mit:
- einem entlang einer Längsachse (x) erstreckten Katheterschaft (2), der einen distalen Endabschnitt (2a) aufweist,
- einem am distalen Endabschnitt (2a) angeordneten proximalen Ballon (3) zum Verankern des Katheterschafts (2) in dem Gefäß (G) sowie zum Aufweiten der Okklusion (O), wobei der proximale Ballon (3) einen Balloninnenraum (30) aufweist, in den zum Entfalten des proximalen Ballons (3) ein fluides Medium einleitbar ist,
- einem am distalen Endabschnitt (2a) angeordneten distalen Ballon (4) zum Aufweiten der Okklusion (O), wobei der distale Ballon (4) einen Balloninnenraum (40) aufweist, in den zum Entfalten des proximalen Ballons (3) ein fluides Medium einleitbar ist, und
- einem vom Katheterschaft (2) umgebenen Führungsdrahtlumen (5) sowie einem im Führungsdrahtlumen (5) führbaren Führungsdraht (500), wobei der Katheterschaft (2) am distalen Endabschnitt (2a) eine Öffnung (20) aufweist, über die der Führungsdraht (500) aus dem Führungsdrahtlumen (5) sowie aus dem Katheterschaft (2) herausführbar ist,
**dadurch gekennzeichnet,**
**dass** das Führungsdrahtlumen den distalen Endabschnitt durchläuft, wobei das Führungsdrahtlumen innerhalb des proximalen Ballons bzw durch den proximalen Ballon, aber nicht den distalen Ballon durchläuft und die Öffnung (20) entlang der Längsachse des Katheterschafts zwischen dem Balloninnenraum des proximalen Ballons und dem Balloninnenraum des distalen Ballons so angeordnet ist, dass der Führungsdraht (500) mit einem über die Öffnung (20) aus dem Führungsdrahtlumen (5) herausgeführten Abschnitt (500a) des Führungsdrahtes (500) außerhalb des distalen Ballons (4) verläuft.

2. Kathetereinrichtung nach Anspruch 1, **dadurch gekennzeichnet, dass** die Kathetereinrichtung (1) ein zu dem Führungsdraht (500) separat ausgebildetes sowie längserstrecktes Führungselement (6) aufweist, das mit einem distalen Endabschnitt (6a) des Führungselements (6) von dem distalen Ballon (4) absteht.

3. Kathetereinrichtung nach Anspruch 2, **dadurch gekennzeichnet, dass** sich das Führungselement (6) durch den Balloninnenraum (40) des distalen Ballons (4) erstreckt, wobei jener distale Endabschnitt (6a) des Führungselements (6) an einem distalen Ende (4a) des distalen Ballons (4) aus dem distalen Ballon (4) herausgeführt ist.

4. Kathetereinrichtung nach Anspruch 2 oder 3, **dadurch gekennzeichnet, dass** das Führungselement (6) durch einen röntgensichtbaren Draht gebildet ist.

5. Kathetereinrichtung nach einem der vorhergehenden Ansprüche, **dadurch gekennzeichnet, dass** ein Durchmesser (D1) des proximalen Ballons (3) quer zur Längsachse (x) des Katheterschafts (2) in einem vollständig entfalteten Zustand des proximalen Ballons (3) mehr als doppelt so groß weiter bevorzugt mehr als 4-fach so groß und am meisten bevorzugt mehr als 10-fach so groß ist als ein Durchmesser (D2) des distalen Ballons (4) quer zur Längsachse (x) des Katheterschafts (2) in einem vollständig entfalteten Zustand des distalen Ballons (4).

6. Kathetereinrichtung nach einem der vorhergehenden Ansprüche, **dadurch gekennzeichnet, dass** das Führungsdrahtlumen (5) des Katheterschafts (2) von einer Wandung (50) umgeben ist.

7. Kathetereinrichtung nach Anspruch 2, **dadurch gekennzeichnet, dass** das Führungselement von der Spitze des distalen Ballons zum aus dem Führungsdrahtlumen herausgeführten Abschnitt, in dem der Führungsdraht außerhalb des distalen Ballons distal zur Öffnung des distalen Endabschnittes verlaufen kann, hingeneigt ist.

8. Kathetereinrichtung nach einem der vorhergehenden Ansprüche, **dadurch gekennzeichnet, dass** der Katheterschaft (2) ein von einer Wandung umgebenes Lumen (7) aufweist, über das zum Entfalten des proximalen und des distalen Ballons (3, 4) ein fluides Medium in den Balloninnenraum (30) des proximalen Ballons (3) und in den Balloninnenraum (40) des distalen Ballons (4) einleitbar ist.

9. Kathetereinrichtung nach Anspruch 8, **dadurch gekennzeichnet, dass** das Lumen (7) in den Balloninnenraum (30) des proximalen Ballons (3) mündet, wobei der Balloninnenraum (40) des distalen Ballons (4) über den Balloninnenraum (30) des proximalen Ballons (3) mit dem Lumen (7) in Strömungsverbindung steht.

10. Kathetereinrichtung nach Anspruch 8, **dadurch gekennzeichnet, dass** sich die Wandung (70) des Lumens (7) durch den Balloninnenraum (30) des proximalen Ballons (3) hindurch erstreckt, wobei das Lumen (7) mit dem Balloninnenraum (40) des distalen Ballons (4) in Strömungsverbindung steht, und wobei die Wandung (70) des Lumens (7) zumindest eine im Balloninnerraum (30) des proximalen Ballons (3) angeordnete laterale Auslassöffnung (71) aufweist, über die ein fluides Medium zum Entfalten des proximalen Ballons (3) in den Balloninnenraum (30) des proximalen Ballons (3) einleitbar ist.

11. Kathetereinrichtung nach Anspruch 2 und nach Anspruch 6 oder einem der Ansprüche 6 bis 11 sofern rückbezogen auf Anspruch 6, **dadurch gekennzeichnet, dass** ein proximaler Endabschnitt (6b) des Führungselements (6) mit der Wandung (50) des Führungsdrahtlumens (5) verbunden ist.

12. Kathetereinrichtung nach den Ansprüchen 2 und nach Anspruch 8 oder einem der Ansprüche 10 bis 11 soweit rückbezogen auf Anspruch 9, **dadurch gekennzeichnet, dass** ein proximaler Endabschnitt (6b) des Führungselements (6) mit der Wandung (70) des Lumens (7) verbunden ist.

13. Kathetereinrichtung nach einem der vorhergehenden Ansprüche, **dadurch gekennzeichnet, dass** das Lumen (7) und das Führungsdrahtlumen (5) nebeneinander im Katheterschaft (2) erstrecken.

14. Kathetereinrichtung nach einem der Ansprüche 1 bis 12, **dadurch gekennzeichnet, dass** das Lumen (7) und das Führungsdrahtlumen (5) koaxial zueinander angeordnet sind, wobei das Führungsdrahtlumen (5) vom Lumen (7) umgeben ist.

15. Kathetereinrichtung nach Anspruch 6 und einem der Ansprüche 7 bis 14 soweit rückbezogen auf Anspruch 6, **dadurch gekennzeichnet, dass** die Kathetereinrichtung (1) mindestens einen Röntgenmarker (8) aufweist, der im Balloninnenraum (30) des proximalen Ballons (3) an der Wandung (50) des Führungsdrahtlumens (5) angeordnet ist.

## Claims

1. A catheter device (1) for recanalizing an occlusion (O) in a vessel (G), comprising:
- a catheter shaft (2) extending along a longitudinal axis (x) and having a distal end section (2a),
- a proximal balloon (3) arranged at the distal end section (2a) for anchoring the catheter shaft (2) in the vessel (G) and for expanding the occlusion (O), wherein the proximal balloon (3) has a balloon interior (30) into which a fluid medium can be introduced for deploying the proximal balloon (3),
- a distal balloon (4) arranged at the distal end section (2a) for expanding the occlusion (O), wherein the distal balloon (4) has a balloon interior (40) into which a fluid medium can be introduced for deploying the proximal balloon (3), and
- a guide wire lumen (5) surrounded by the catheter shaft (2) and a guide wire (500) which can be guided in the guide wire lumen (5), wherein the catheter shaft (2) has an opening (20) at the distal end section (2a), via which the guide wire (500) can be guided out of the guide wire lumen (5) and out of the catheter shaft (2),
**characterized in**
**that** the guide wire lumen passes through the distal end section, wherein the guide wire lumen passes inside the proximal balloon or through the proximal balloon but not through the distal balloon and the opening (20) is arranged along the longitudinal axis of the catheter shaft between the balloon interior of the proximal balloon and the balloon interior of the distal balloon in such a way that the guide wire (500) extends with a section (500a) of the guide wire (500) led out of the guide wire lumen (5) via the opening (20) outside the distal balloon (4).

2. The catheter device according to claim 1, **characterized in that** the catheter device (1) has a guide element (6) which is configured separately from the guide wire (500) and is longitudinally extended and which projects from the distal balloon (4) with a distal end section (6a) of the guide element (6).

3. The catheter device according to claim 2, **characterized in that** the guide element (6) extends through the balloon interior (40) of the distal balloon (4), wherein that distal end section (6a) of the guide element (6) is led out of the distal balloon (4) at a distal end (4a) of the distal balloon (4).

4. The catheter device according to claim 2 or 3, **characterized in that** the guide element (6) is formed by an X-ray visible wire.

5. The catheter device according to one of the preceding claims, **characterized in that** a diameter (D1) of the proximal balloon (3) transverse to the longitudinal axis (x) of the catheter shaft (2) in a fully deployed state of the proximal balloon (3) is more than twice as large, further preferably more than 4 times as large and most preferably more than 10 times as large as a diameter (D2) of the distal balloon (4) transverse to the longitudinal axis (x) of the catheter shaft (2) in a fully deployed state of the distal balloon (4).

6. The catheter device according to one of the preceding claims, **characterized in that** the guide wire lumen (5) of the catheter shaft (2) is surrounded by a wall (50).

7. The catheter device according to claim 2, **characterized in that** the guide element is inclined from the tip of the distal balloon to the section extending out of the guide wire lumen, in which the guide wire can run outside the distal balloon distal to the opening of the distal end section.

8. The catheter device according to one of the preceding claims, **characterized in that** the catheter shaft (2) has a lumen (7) which is surrounded by a wall and via which a fluid medium can be introduced into the balloon interior (30) of the proximal balloon (3) and into the balloon interior (40) of the distal balloon (4) in order to deploy the proximal and distal balloons (3, 4).

9. The catheter device according to claim 8, **characterized in that** the lumen (7) opens into the balloon interior (30) of the proximal balloon (3), wherein the balloon interior (40) of the distal balloon (4) is in flow communication with the lumen (7) via the balloon interior (30) of the proximal balloon (3).

10. The catheter device according to claim 8, **characterized in that** the wall (70) of the lumen (7) extends through the balloon interior (30) of the proximal balloon (3), wherein the lumen (7) is in flow communication with the balloon interior (40) of the distal balloon (4), and wherein the wall (70) of the lumen (7) has at least one lateral outlet opening (71) arranged in the balloon interior (30) of the proximal balloon (3), via which a fluid medium for deploying the proximal balloon (3) can be introduced into the balloon interior (30) of the proximal balloon (3).

11. The catheter device according to claim 2 and according to claim 6 or one of claims 6 to 11, provided that referring back to claim 6, **characterized in that** a proximal end section (6b) of the guide element (6) is connected to the wall (50) of the guide wire lumen (5).

12. The catheter device according to claims 2 and according to claim 8 or one of claims 10 to 11 insofar as referring back to claim 9, **characterized in that** a proximal end section (6b) of the guide element (6) is connected to the wall (70) of the lumen (7).

13. The catheter device according to one of the preceding claims, **characterized in that** the lumen (7) and the guidewire lumen (5) extend side by side in the catheter shaft (2).

14. The catheter device according to one of claims 1 to 12, **characterized in that** the lumen (7) and the guidewire lumen (5) are arranged coaxially to each other, wherein the guidewire lumen (5) is surrounded by the lumen (7).

15. The catheter device according to claim 6 and one of claims 7 to 14 insofar as related back to claim 6, **characterized in that** the catheter device (1) has at least one X-ray marker (8) which is arranged in the balloon interior (30) of the proximal balloon (3) on the wall (50) of the guidewire lumen (5).

## Revendications

1. Dispositif de cathéter (1) permettant de recanaliser une occlusion (O) dans un vaisseau sanguin (G), comprenant :
- une tige de cathéter (2) s'étendant le long d'un axe longitudinal (x) et présentant une section d'extrémité distale (2a),
- un ballonnet proximal (3) agencé au niveau de la section d'extrémité distale (2a) et permettant d'ancrer la tige de cathéter (2) dans le vaisseau sanguin (G), et permettant d'élargir l'occlusion (O), le ballonnet proximal (3) présentant un espace intérieur de ballonnet (30) au sein duquel un milieu fluide peut être introduit afin de déployer le ballonnet proximal (3),
- un ballonnet distal (4) agencé au niveau de la section d'extrémité distale (2a) et permettant d'élargir l'occlusion (O), le ballonnet distal (4) présentant un espace intérieur de ballonnet (40) au sein duquel un milieu fluide peut être introduit afin de déployer le ballonnet proximal (3), et
- une lumière de fil-guide (5) entourée par la tige de cathéter (2), ainsi qu'un fil-guide (500) pouvant être guidé dans la lumière de fil-guide (5), la tige de cathéter (2) présentant au niveau de la section d'extrémité distale (2a) un orifice (20) par l'intermédiaire duquel le fil-guide (500) peut être guidé hors de la lumière de fil-guide (5) et hors de la tige de cathéter (2),
**caractérisé en ce que**
la lumière de fil-guide traverse la section d'extrémité distale, la lumière de fil-guide passant à l'intérieur du ballonnet proximal ou à travers le ballonnet proximal, mais pas à travers le ballonnet distal, et l'orifice (20) étant agencé le long de l'axe longitudinal de la tige de cathéter entre l'espace intérieur de ballonnet du ballonnet proximal et l'espace intérieur de ballonnet du ballonnet distal de façon telle que le fil-guide (500), par une section (500a) du fil-guide (500) guidée hors de la lumière de fil-guide (5) via l'orifice (20), s'étend à l'extérieur du ballonnet distal (4).

2. Dispositif de cathéter selon la revendication 1, **caractérisé en ce que** le dispositif de cathéter (1) présente un élément de guidage (6) allongé et réalisé de manière séparée par rapport au fil-guide (500) qui fait saillie depuis le ballonnet distal (4) par une section d'extrémité distale (6a) de l'élément de guidage (6).

3. Dispositif de cathéter selon la revendication 2, **caractérisé en ce que** l'élément de guidage (6) s'étend à travers l'espace intérieur de ballonnet (40) du ballonnet distal (4), la section d'extrémité distale (6a) de l'élément de guidage (6) étant guidée hors du ballonnet distal (4) au niveau d'une extrémité distale (4a) du ballonnet distal (4).

4. Dispositif de cathéter selon la revendication 2 ou 3, **caractérisé en ce que** l'élément de guidage (6) est formé par un fil visible aux rayons X.

5. Dispositif de cathéter selon l'une des revendications précédentes, **caractérisé en ce qu'**un diamètre (D1) du ballonnet proximal (3) transversalement à l'axe longitudinal (x) de la tige de cathéter (2), dans un état complètement déployé du ballonnet proximal (3), est plus de deux fois plus grand, de manière plus préférée plus de 4 fois plus grand, et de la manière la plus préférée plus de 10 fois plus grand, qu'un diamètre (D2) du ballonnet distal (4) transversalement à l'axe longitudinal (x) de la tige de cathéter (2) dans un état complètement déployé du ballonnet distal (4).

6. Dispositif de cathéter selon l'une des revendications précédentes, **caractérisé en ce que** la lumière de fil-guide (5) de la tige de cathéter (2) est entourée par une paroi (50).

7. Dispositif de cathéter selon la revendication 2, **caractérisé en ce que** l'élément de guidage est incliné depuis la pointe du ballonnet distal jusqu'à la section qui est guidée hors de la lumière de fil-guide et au sein de laquelle le fil-guide peut s'étendre à l'extérieur du ballonnet distal de manière distale par rapport à l'orifice de la section d'extrémité distale.

8. Dispositif de cathéter selon l'une des revendications précédentes, **caractérisé en ce que** la tige de cathéter (2) présente une lumière (7) entourée par une paroi, par laquelle un milieu fluide peut être introduit dans l'espace intérieur de ballonnet (30) du ballonnet proximal (3) et dans l'espace intérieur de ballonnet (40) du ballonnet distal (4) afin de déployer les ballonnets proximal et distal (3, 4).

9. Dispositif de cathéter selon la revendication 8, **caractérisé en ce que** la lumière (7) débouche dans l'espace intérieur de ballonnet (30) du ballonnet proximal (3), l'espace intérieur de ballonnet (40) du ballonnet distal (4) étant en communication d'écoulement avec la lumière (7) par l'intermédiaire de l'espace intérieur de ballonnet (30) du ballonnet proximal (3).

10. Dispositif de cathéter selon la revendication 8, **caractérisé en ce que** la paroi (70) de la lumière (7) s'étend à travers l'espace intérieur de ballonnet (30) du ballonnet proximal (3), la lumière (7) étant en communication d'écoulement avec l'espace intérieur de ballonnet (40) du ballonnet distal (4), et la paroi (70) de la lumière (7) présentant au moins un orifice de sortie latéral (71) agencé dans l'espace intérieur de ballonnet (30) du ballonnet proximal (3) et par l'intermédiaire duquel un milieu fluide peut être introduit dans l'espace intérieur de ballonnet (30) du ballonnet proximal (3) afin de déployer le ballonnet proximal (3).

11. Dispositif de cathéter selon la revendication 2 et selon la revendication 6 ou l'une des revendications 6 à 11 lorsqu'elle dépend de la revendication 6, **caractérisé en ce qu'**une section d'extrémité proximale (6b) de l'élément de guidage (6) est reliée à la paroi (50) de la lumière de fil-guide (5).

12. Dispositif de cathéter selon la revendication 2 et selon la revendication 8 ou l'une des revendications 10 à 11 lorsqu'elle dépend de la revendication 9, **caractérisé en ce qu'**une section d'extrémité proximale (6b) de l'élément de guidage (6) est reliée à la paroi (70) de la lumière (7).

13. Dispositif de cathéter selon l'une des revendications précédentes, **caractérisé en ce que** la lumière (7) et la lumière de fil-guide (5) s'étendent côte à côte dans la tige de cathéter (2).

14. Dispositif de cathéter selon l'une des revendications 1 à 12, **caractérisé en ce que** la lumière (7) et la lumière de fil-guide (5) sont agencées de manière coaxiale l'une par rapport à l'autre, la lumière de fil-guide (5) étant entourée par la lumière (7).

15. Dispositif de cathéter selon la revendication 6 et l'une des revendications 7 à 14 lorsqu'elle dépend de la revendication 6, **caractérisé en ce que** le dispositif de cathéter (1) présente au moins un marqueur à rayons X (8) qui est agencé dans l'espace intérieur de ballonnet (30) du ballonnet proximal (3), au niveau de la paroi (50) de la lumière de fil-guide (5).
